# EUROPEAN PATENT APPLICATION

(11) **EP 4 349 347 A1**
(43) Date of publication of application: **10.04.2024**
(21) Application number: 22799128.8
(22) Date of filing: 04.05.2022
(51) Int. Cl.: A61K 35/28, A61P 9/10, A61P 17/02, A61P 17/14, A23L 33/10, C12N 5/0775, A61K 31/7105, A61K 38/18

(54) **COMPOSITION FOR PROMOTING ANGIOGENESIS COMPRISING EXTRACELLULAR VESICLES DERIVED FROM THREE-DIMENSIONAL SPHEROID-TYPE CELL AGGREGATE**

(30) Priority: 04.05.2021 KR 20210058172
(71) Applicant: S&E Bio Co., Ltd., Seoul 06351 (KR)
(72) Inventor: KIM, Eun Hee, Seoul 06608 (KR); SUNG, Ji Hee, Seoul 08716 (KR); SHIN, Eun Kyoung, Seoul 07611 (KR)
(74) Representative: Potter Clarkson
(86) International application number: PCT/KR2022/006423
(87) International publication number: WO 2022/235085

(57) **Abstract**

The present invention relates to: a composition for promoting angiogenesis, comprising extracellular vesicles derived from a three-dimensional spheroid-type cell aggregate that are produced using a novel production method; and a method for promoting angiogenesis. The extracellular vesicles produced by a novel method of the present invention highly express various factors capable of promoting angiogenesis and can induce the regeneration of blood vessels, and thus can be effectively used in the treatment of various diseases that require vascular regeneration.

## Description

### [Technical Field]

The present invention relates to a composition for promoting angiogenesis, including extracellular vesicles derived from a three-dimensional spheroid-type cell aggregate that are produced using a novel production method, and a method for promoting angiogenesis.

### [Background Art]

Treatments using stem cells, particularly mesenchymal stem cells (MSCs), and positive clinical results have been reported for various diseases. However, stem cell therapy has a risk of cell-related side effects such as vascular occlusion, tumor formation, and coagulation disorders, and efficacy verification through clinical trials is still required. It is known that a paracrine effect of stem cells induces the promotion of the regeneration and vascular regeneration of surrounding skin cells, and in particular, extracellular vesicles (EV) are known as a major efficacious factor in the paracrine effect.

The extracellular vesicles are classified into exosomes and microvesicles depending on a size, and the exosomes have a diameter of 30 to 150 nm, and the microvesicles have a size of 100 to 1,000 nm. The extracellular vesicles are parts of the cell membrane that have been separated into the blood, and are known to mediate intercellular communication by containing both protein and nuclear components. Using extracellular vesicles instead of stem cells not only increases safety by minimizing the side effects by using stem cells, but also is advantageous in terms of biodistribution and production process.

However, mass production, obtaining method, and the like for using extracellular vesicles derived from stem cells have not yet been established, and much research has been not conducted on methods to further enhance the efficacy of extracellular vesicles while maintaining the characteristics of stem cell-derived extracellular vesicles.

Accordingly, there is a need for extracellular vesicles with improved efficacy and novel therapeutic agents using the same.

### [Disclosure]

### [Technical Problem]

Therefore, the present inventors studied a method capable of shortening a culture time while enabling mass production, in order to produce three-dimensional spheroid-type cell aggregates or extracellular vesicles derived therefrom. As a result, the present inventors confirmed that extracellular vesicles with improved angiogenesis promotion were produced by producing a three-dimensional spheroid-type cell aggregate (3D-static-spheroid) through static culture using a microwell and producing extracellular vesicles using the same, and then completed the present invention.

Accordingly, an object of the present invention is to prepare extracellular vesicles derived from a three-dimensional spheroid-type cell aggregate with improved angiogenesis promotion and to provide a composition for promoting angiogenesis including the same.

### [Technical Solution]

An aspect of the present invention provides a pharmaceutical composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Another aspect of the present invention provides a food composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Yet another aspect of the present invention provides an *in vitro* composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Yet another aspect of the present invention provides a method for producing a composition for promoting angiogenesis including extracellular vesicles derived from a three-dimensional spheroid-type cell aggregate, including (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Yet another aspect of the present invention provides a method for promoting angiogenesis including (a) preparing a three-dimensional spheroid-type cell aggregate by 3-dimensionally (3D) culturing stem cells in a microwell with a diameter of 200 to 800 µm and a depth of 100 to 1000 µm; (b) preparing extracellular vesicles derived from the three-dimensional spheroid-type cell aggregate by isolating extracellular vesicles from the three-dimensional spheroid-type cell aggregate; and (c) treating the extracellular vesicles derived from the three-dimensional spheroid-type cell aggregate prepared in step (b) to a subject in need thereof.

### [Advantageous Effects]

The extracellular vesicles produced by a novel method of the present invention highly express various factors capable of promoting angiogenesis and can induce the regeneration of blood vessels, and thus can be effectively used in the treatment of various diseases that require vascular regeneration.

### [Description of Drawings]

FIG. 1 is a schematic diagram illustrating a process of producing a 3D spheroid-type cell aggregate and isolating extracellular vesicles therefrom.
FIG. 2A is a diagram of observing a microwell containing a 3D-dynamic-PEG spheroid culture medium.
FIG. 2B is a diagram of observing a microwell containing a 3D-static-spheroid culture medium.
FIG. 2C is a diagram illustrating changes in aggregate area according to culture periods of 3D-dynamic-PEG spheroid and 3D-static-spheroid produced using a microwell.
FIG. 3 is a diagram of comparing size distributions of 3D-static-spheroid and 3D-dynamic-PEG spheroid.
FIG. 4 is a diagram of observing the shape of 3D-static-spheroid EVs with an electron microscope.
FIG. 5 is a diagram illustrating results of nanoparticle tracking analysis (NTA) to confirm the concentration and size distribution of 3D-static-spheroid EV.
FIG. 6 is a diagram illustrating results of ELISA and Western blot to confirm expression markers of 3D-static-spheroid EV.
FIG. 7 is a diagram of comparing productions of 3D-static-spheroid EV, 3D-dynamic-PEG spheroid EV, and 2D-EV per derived cell.
FIG. 8 is a diagram illustrating miRNAs and proteins highly expressed in 3D-static-spheroid EV compared to 2D-EV.
FIG. 9 is a diagram illustrating angiogenesis-related miRNAs highly expressed in 3D-static-spheroid EV compared to 3D-dynamic-PEG spheroid EV.
FIG. 10 is a diagram illustrating a result of confirming GAPDH and an effect on Ephrin A3 expression inhibition in HUVEC cells according to treatment of 2D-EV and 3D-static-spheroid EV; and a result of comparing the expression of angiogenesis-related miRNA, miR-210 in 2D-EV and 3D-static-spheroid EV.
FIG. 11A is a diagram illustrating a result of confirming differences in EV production, EV size, and EV-containing protein amount depending on a donor in 2D-EV and 3D-static-spheroid EV (WJ-3D EV).
FIG. 11B is a diagram illustrating a result of confirming donor variation and differences in expression level of angiogenesis-related miRNA in 2D-cultured WJ-MSC, 3D-cultured WJ-MSC, 2D-EV, and 3D-static-spheroid EV.
FIG. 12 is a diagram illustrating a result of confirming changes in expression of angiogenesis-related factors, VEGF, Hif-1a, and FGF, after treating vascular endothelial cells (HUVECs) with 3D-static-spheroid EV.
FIG. 13 is a diagram illustrating a result of treating vascular endothelial cells (HUVECs) with PBS (con), VEGF, 2D-EV, and 3D-static-spheroid EV and comparing tube formation effects.
FIG. 14 is a diagram illustrating a result of confirming changes in volumes of ventricles and cerebral infarction lesions after treating a cerebral infarction animal model with PBS as a control group or 3D-static-spheroid EV as an experimental group.
FIG. 15 is a diagram illustrating a result of confirming a neoangiogenic effect, after treating a cerebral infarction animal model with PBS as a control group or 3D-static-spheroid EV as an experimental group.
FIG. 16 is a diagram illustrating a result of confirming an effect of restoring motor function loss, after injecting 3D-static-spheroid EVs into a cerebral infarction animal model.
FIG. 17 is a diagram illustrating a result of confirming the size, roundness, and solidity of 3D spheroids produced after varying the diameter and depth of a microwell and the number of cells per well in the production of 3D-static spheroid EV.
FIG. 18 is a diagram illustrating a result of confirming the expression levels of miRNA-132 and miRNA-210 expressed in 3D-static-spheroid EV and 2D-EV prepared under various conditions.
FIG. 19 is a diagram illustrating a result of confirming angiogenesis ability of 3D-static-spheroid EV and 2D-EV prepared under various conditions through a tube formation experiment.

### [Best Mode for the Invention]

The present invention provides a pharmaceutical composition for promoting angiogenesis including extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

In the present invention, the cells may be used without limitation as long as the cells are cells capable of isolating extracellular vesicles, and may be cells isolated from a natural organism. In addition, the cells may be derived from any type of animal or plant, including humans and non-human mammals, and may be various types of immune cells, tumor cells, and stem cells, and preferably, the stem cells may be mesenchymal stem cells, pluripotent stem cells, induced pluripotent stem cells, or embryonic stem cells.

In the present invention, the 3D culture means culturing in a three-dimensional arrangement *in vitro,* and unlike 2D culture, the cell growth in the 3D culture may grow cells in all directions *in vitro* and may be more similar to an *in vivo* cell environment.

In the present invention, the 3D culture in step (a) may be performed by any 3D cell culture technique known in the art to which the present invention pertains, and may be a cell culture using, for example, microwell array culture, porous microsphere culture, hanging drop culture, low attachment plate culture, membrane-based cell-detachment culture, thermal lifting culture, centrifugation culture, semisolid medium culture, and the like. Preferably, the 3D culture may be dynamic culture or static culture, more preferably static culture. In the present invention, when the 3D culture of step (a) is static culture, it makes it easier to culture without requiring devices necessary for shaking culture, and mass-culture is enabled in the Good Manufacturing Practices (GMP) manufacturing place.

In the present invention, the 3D culture in step (a) may be performed for 1 to 10 days, preferably 2 to 4 days. In the present invention, when the culture in step (a) is performed for 2 to 4 days, the viability of cells present in the three-dimensional spheroid-type cell aggregate is maintained at a high level, and compared to a conventional process of producing a three-dimensional spheroid-type cell aggregate, the culture time is relatively short, so that it is possible to rapidly produce a three-dimensional spheroid-type cell aggregate and extracellular vesicles derived therefrom.

In the present invention, the 3D culture in step (a) may be performed by dispensing mesenchymal stem cells in a microwell at a density of 100 to 1000 cells/well, preferably a density of 100 to 600 cells/well, more preferably a density of 100 to 500 cells/well.

In the present invention, in the isolating of the extracellular vesicles in step (b), a sample containing cells or a cell aggregate may be prepared by using a method selected from the group consisting of extrusion, sonication, cell lysis, homogenization, freeze-thawing, electroporation, chemical treatment, mechanical degradation, and treatment of physical stimuli externally applied to cells, preferably isolated by a tangential flow filtration (TFF) method, but is not limited thereto.

In the present invention, microRNA is named by attaching "mir" in the front and adding "-" and a number at the end. At this time, the numbers often indicate the order to be named, and for example, mir-123 was named before mir-156 and is expected to have been identified earlier. "mir-" indicates pre-microRNA, and capitalized "miR" refers to mature microRNA. Except for one or two sequences, microRNAs with almost identical sequences are named by adding small letters. For example, miR-121a and miR-121b were generated from respective precursors mir-121a and mir-121b, and their sequences are very similar. Mature microRNAs are identical, but pre-microRNAs located at different sites on a genome are named by adding "-" and a number. For example, the pre-microRNAs mir-121-1 and mir-121-2 become the same mature microRNA (miR-121), but are located at different sites on the genome. Species-specific names of microRNAs are indicated in the front. For example, hsa-miR-123 is Homo sapiens microRNA, and oar-miR-123 is Ovis aries microRNA. 'v' means viral (miRNA encoded by a viral genome) and 'd' means Drosophila microRNA. When two mature microRNAs are derived from different arms (3' arm or 5' arm) of the same pre-microRNA, the mature microRNAs are named by adding `-3p' or '-5p' to the end. miR-142-3p is derived from the 3' arm, and miR-142-5p is derived from the 5' arm. The nomenclature of MicroRNA generally follows the above criteria, but there are exceptions.

In the present invention, the extracellular vesicles may highly express clinically significant substances compared to known extracellular vesicles, and the clinically significant substances may be substances exhibiting an angiogenesis effect. For example, preferably, the extracellular vesicles of the present invention may highly express at least one selected from the group consisting of angiogenesis-related miR-27a, miR-132, miR-146a, miR-146b, miR-184, and miR-210, and may highly express at least one selected from the group consisting of a vascular endothelial growth factor (VEGF), a hypoxia-inducible factor 1-alpha (Hif-1a) and a fibroblast growth factor (FGF) compared to extracellular vesicles derived from a spheroid-type cell aggregate obtained by 3D dynamic culture of mesenchymal stem cells and extracellular vesicles derived from 2D-cultured mesenchymal stem cells. More preferably, the extracellular vesicles of the present invention may highly express angiogenesis-related miR-27a, miR-132, miR-146a, miR-146b, miR-184, and miR-210, VEGF, Hif-1a and FGF compared to extracellular vesicles derived from a spheroid-type cell aggregate obtained by 3D dynamic culture of mesenchymal stem cells and extracellular vesicles derived from 2D-cultured mesenchymal stem cells.

In addition, the extracellular vesicles may be incorporated and internalized into cells upon treatment with cells, and when incorporated and internalized into cells, a clinically significant substance highly expressed in the extracellular vesicles may be effectively delivered to cells to be highly expressed in cells.

In the present invention, the extracellular vesicles derived from the three-dimensional spheroid-type cell aggregates that exhibit an angiogenesis-promoting effect may be used interchangeably with "3D-static-spheroid-EVs". In addition, in comparison, the extracellular vesicles derived from the 3D dynamically cultured spheroid-type cell aggregate may be used interchangeably with "3D-dynamic-PEG-spheroid-EVs".

In the present invention, the "extracellular vesicles derived from the spheroid-type cell aggregate obtained by 3D dynamic culture of mesenchymal stem cells" are any extracellular vesicles isolated from a spheroid-type cell aggregate obtained by 3D dynamic culture of mesenchymal stem cells without limitation, preferably extracellular vesicles disclosed in Registration Patent (application No. 10-2016-0053026, method for producing stem cell-derived extracellular vesicles).

In the present invention, the "extracellular vesicles derived from 2D-cultured mesenchymal stem cells" may be any extracellular vesicles obtained by culturing stem cells according to a conventional 2D culture method and isolated from the stem cells by a conventional method of isolating extracellular vesicles without limitation. In an embodiment of the present invention, the conventional 2D culture method was performed by a method of washing stem cells cultured for 3 days in a cell stack with PBS, replacing the medium with a serum-free medium, and further culturing the cells for 2 days.

In the present invention, the three-dimensional spheroid-type cell aggregate may have an average diameter of 74.43 ± 7.756 µm, preferably a size range of 55 to 95.0 µm. As a result of measuring the size distribution of 155 three-dimensional spheroid-type cell aggregates, the average diameter may be 74.43 µm and the coefficient of variance (CV) may be 9.59%. The three-dimensional spheroid-type cell aggregate of the present invention may have a higher kurtosis in size distribution than the "spheroid-type cell aggregate obtained by 3D dynamic culture of mesenchymal stem cells". Therefore, the size of the three-dimensional spheroid-type cell aggregate is smaller than that of the "spheroid-type cell aggregate obtained by 3D dynamic culture of mesenchymal stem cells" and may have a relatively uniform size distribution.

In the present invention, the microwell may be coated with any one selected from the group consisting of TMSPMA (3-(Trimetoxysily) propylmethacrylate), HEA (Hydroxyethyl acrylate), GMA (Glycidyl methacrylate), EGDMA (diethyleneglycol dimethacrylate), THFA (Tetrahydrofurfuryl acrylate), HMAA (Hydroxymethul acrylamide), PEA (Phenyl epoxyacrylate), HOFHA (6-Hydroxy-2, 2,3,3,4,4,5,5-octafluoro), EOPT (Polyethoxylated(4)pentaerythritoltetraacrylate), HPA (Hydroxypropyl acrylate), BMA (Buthylmethacrlate), PETIA (Pentaerythritol triacrylate), HDDA (Hexan diol diacrylate), EGPEA (Ethyleneglycol phenyletheracrylate), BM (Benzylmethacrylate), HPPA (Hydroxyphenoxypropyl acrylate), BHPEA (2-(4-Benzoyl-3-hydroxyphenoxy)ethylacrylate), HEMA (Hydroxyethyl methacrylate), HPMA (N-(2-Hydroxypropyl) methacrylamide) and MPC (2-Methacryloyloxyethyl Phosphorylcholine Polymer), and preferably, coated with MPC (2-Methacryloyloxyethyl Phosphorylcholine Polymer), but is not limited thereto.

The microwell of the present invention may have a diameter of 200 to 800 µm, preferably 300 to 800 µm, and more preferably 400 to 800 µm.

In addition, the microwell may be a microwell with a flat structure without depth, or in the case of a microwell with a depth, the microwell may have a structure of 100 to 1000 µm, preferably 100 to 900 µm, and more preferably 200 to 900 µm.

The mesenchymal stem cells cultured by the structure may maintain a high survival rate even after the culture time has elapsed. Preferably, the production yield of cell aggregates may be increased by manufacturing a microarray including 1,000 to 100,000 microwells.

When the extracellular vesicles are produced by the "method for producing the extracellular vesicles derived from the three-dimensional spheroid-type cell aggregate" of the present invention, in addition to the manufacturing advantages of static culture, extracellular vesicles with improved clinical applicability, especially angiogenesis ability, may be mass-produced quickly and efficiently. In particular, existing methods for producing extracellular vesicles, such as a method for producing "extracellular vesicles derived from a spheroid-type cell aggregate obtained by 3D dynamic-culturing of mesenchymal stem cells" or a method of producing "extracellular vesicles derived from mesenchymal stem cells 2D cultured", are unsuitable for GMP. However, the method for producing extracellular vesicles of the present invention has a characteristic of being suitable for GMP application to be particularly suitable for producing a pharmaceutical composition for angiogenesis.

In the present invention, the angiogenesis refers to the formation of new blood vessels and means a process of inducing or increasing the migration of vascular endothelial cells and promoting tube formation in the vascular endothelial cells to form new blood vessels from existing blood vessels.

The extracellular vesicles derived from the three-dimensional spheroid-type cell aggregate of the present invention highly express various factors capable of promoting angiogenesis and may induce the regeneration of blood vessels, and thus can be effectively used in the treatment of various diseases that require vascular regeneration.

The types of diseases requiring angiogenesis to which the extracellular vesicles derived from the three-dimensional spheroid-type cell aggregate of the present invention may be applied are not limited thereto but may be at least one selected from the group consisting of burns, ulcers, ischemia, arteriosclerosis, angina pectoris, myocardial infarction, cardiovascular diseases, cerebrovascular diseases, and alopecia, particularly cardiovascular diseases, or cerebrovascular diseases.

In addition, since the extracellular vesicles derived from the three-dimensional spheroid-type cell aggregate of the present invention are particularly useful in promoting brain angiogenesis, the composition for promoting angiogenesis may be used to promote brain angiogenesis.

Brain tissue normally receives a large amount of blood flow, but when cerebral blood vessels are blocked due to external or internal stimulation, the amount of blood supplied to the brain decreases, so that the brain tissue is not properly functioning. If a decrease in cerebral blood flow continues for a certain period of time or more, brain tissue is necrotized, but the brain tissue is necrotized to reach an irreversible state, which is called cerebral infarction, including cerebral thrombosis and cerebral embolism. Thrombosis refers to blocking the blood vessel by blood clotting in the local area of a blood vessel, and when a blood clot occurs, blood flow is cut off in an area of the blood vessel, and if compensation by a collateral pathway from elsewhere is not sufficient, brain tissue in the area dies. The embolism refers to blocking peripheral blood vessels when a blood clot formed in the heart is transported to the periphery by the bloodstream, and when blood vessels are blocked by emboli, the brain tissue in the area dies. The extracellular vesicles derived from the three-dimensional spheroid-type cell aggregate of the present invention may promote the brain angiogenesis, and thus may be useful in restoring decreased cerebral blood flow caused by cerebral infarction.

The pharmaceutical composition of the present invention may further include suitable carriers, excipients, and diluents which are commonly used in the preparation of pharmaceutical composition in addition to the active ingredient. The pharmaceutical composition of the present invention may further include other pharmaceutically active ingredients or active mixtures.

The pharmaceutical composition of the present invention may be formulated and used in the form of oral formulations such as powders, granules, tablets, capsules, suspensions, emulsions, syrups, aerosols, etc., external preparations, suppositories, and sterile injectable solutions according to conventional methods, respectively. The carrier, the excipient, and the diluent that may be included in the composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. When the pharmaceutical composition is formulated, the formulation may be prepared by using diluents or excipients, such as a filler, an extender, a binder, a wetting agent, a disintegrating agent, and a surfactant, which are generally used. Solid formulations for oral administration include tablets, pills, powders, granules, capsules, and the like, and these solid formulations may be prepared by mixing at least one or more excipients, for example, starch, calcium carbonate, sucrose or lactose, gelatin, and the like. Further, lubricants such as magnesium stearate and talc are used in addition to simple excipients.

Liquid formulations for oral administration may correspond to suspensions, oral liquids, emulsions, syrups, and the like, and may include various excipients, for example, a wetting agent, a sweetener, an aromatic agent, a preservative, and the like, in addition to water and liquid paraffin which are commonly used as simple diluents. Formulations for parenteral administration include sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base compound of the suppository, witepsol, macrogol, tween 61, cacao butter, laurinum, glycerogelatin, and the like may be used.

A preferable dose of the pharmaceutical composition of the present invention varies according to the condition and weight of a patient, the degree of a disease, a drug form, and the route and period of administration, but may be properly selected by those skilled in the art. The administration may be performed once a day or several times a day. The dose does not limit the scope of the present invention in any aspect.

The pharmaceutical composition of the present invention may be administered to mammals such as mice, rats, livestock, and humans through various routes. All methods of administration may be expected, and for example, the pharmaceutical composition may be administered by oral, rectal or intravenous, intramuscular, subcutaneous, intrauterine dural or intracerebroventricular injection.

The definitions of terms for the excipients, binders, disintegrants, lubricants, flavor enhancers, flavoring agents, and the like of the present invention are described in literature known in the art and include those with the same or similar functions.

Further, the present invention provides a method for promoting angiogenesis including (a) preparing a three-dimensional spheroid-type cell aggregate by 3-dimensionally (3D) culturing stem cells in a microwell with a diameter of 200 to 800 µm and a depth of 100 to 1000 µm; and (b) preparing extracellular vesicles derived from the three-dimensional spheroid-type cell aggregate by isolating extracellular vesicles from the three-dimensional spheroid-type cell aggregate; and (c) treating the extracellular vesicles derived from the three-dimensional spheroid-type cell aggregate prepared in step (b) to a subject in need thereof.

The subject is preferably mammals, including humans, and patients in need of angiogenesis include all patients being treated, patients who have previously received treatment, and patients in need of treatment for diseases, and may also include patients who have undergone surgical procedures for angiogenesis.

Additionally, the present invention may be treated in combination with drugs or treatment methods for angiogenesis. When the present invention is used in combination, the present invention may be treated simultaneously or sequentially with other drugs or treatment methods for angiogenesis.

Further, the present invention provides a food composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

The food composition may be cited in the same manner as the description of the pharmaceutical composition. The foods especially include health functional foods. The "health functional food" defined in the present invention refers to food produced and processed using raw materials or ingredients with functionality, which are useful for the human body, and the "functionality" means intake for adjusting nutrients for the structures and functions of the human body or obtaining a useful effect on health applications such as physiological actions. The health functional food may have any one form of tablets, capsules, powders, granules, liquids, and pills.

In addition, the food composition of the present invention may be a food composition in which functional ingredients are added to various foods or beverages. The food may have any one form of, for example, beverages, powdered beverages, solids, chewing gum, tea, vitamin complexes, and food additives.

Further, the present invention provides an *in vitro* composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

The *in vitro* composition for promoting angiogenesis of the present invention may be used for experimental purposes and may be a composition for treating isolated cells or tissues requiring angiogenesis. The *in vitro* composition for promoting angiogenesis of the present invention may be a medium composition containing the extracellular vesicles derived from the three-dimensional spheroid-type cell aggregate, and the medium may include, without limitation, any medium known to those skilled in the art, such as a medium containing serum (e.g., fetal bovine serum, horse serum, and human serum). Media that may be used in the present invention may include, for example, RPMI series, EMEM, MEM, Iscove's MEM, 199 medium, CMRL 1066, RPMI 1640, F12, F10, DMEM, a mixture of DMEM and F12, Way-mo, McCoy's 5A, or media known in the art suitable for culturing cells requiring angiogenesis.

Further, the present invention provides a method for producing a composition for promoting angiogenesis including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

According to the producing method, the extracellular vesicles derived from the three-dimensional spheroid-type cell aggregate, which have an excellent angiogenesis promoting effect, may be quickly mass-produced in accordance with GMP standards.

Duplicated contents are omitted in consideration of the complexity of the present specification, and terms not defined otherwise in the present specification have the meanings commonly used in the art to which the present invention pertains.

Hereinafter, the present invention will be described in detail by Examples. However, the following Examples are just illustrative of the present invention, and the contents of the present invention are not limited to the following Examples.

### [Modes for the Invention]

### Western blot

Cells and extracellular vesicles were dissolved in a radioimmunoprecipitation assay (RIPA) buffer (25 mM Tris-HCl, pH 7.6, 150 mM NaCl, 0.5% triton X-100, 1% Na-deoxycholate, 0.1% sodium dodecyl sulfate (SDS) and protease inhibitor). A total of 20 µg of proteins was isolated by SDS-polyacrylamide gel electrophoresis and transferred to a nitrocellulose membrane (Bio-Rad, Hercules, CA, USA). Thereafter, the nitrocellulose membrane was cultured overnight at 4°C together with a primary antibody against histone H2A.Z, histone H3, lamin A/C, flotillin-1 (1 : 1,000, Cell signaling technology, Beverly, MA, USA) or calreticulin (1 : 1,000, ThermoFisher Scientific, Inc., Rockford, IL, USA). After washed with Tris-buffered saline-Tween 20, the nitrocellulose membrane was cultured for 2 hours together with a horseradish peroxidase (HRP)-conjugated secondary antibody (1 : 1,000, anti-rabbit, Cell Signaling Technology, Beverly, MA, USA). The proteins were detected using a chemiluminescent substrate from ThermoFisher Scientific, Inc. (Waltham, MA, USA). Labeled proteins were visualized via an X-ray film (Agfa, Mortsel, Belgium).

### ELISA

ELISA was performed with a commercial kit according to the manual of an individual manufacturer. The following ELISA kits were used: gentamicin (5111GEN, EuroProxima, Arnhem, Nederland), bovine albumin (8100, Alpha Diagnostic, San Antonio, TX, USA), Hsp70 (Abcam, Cambridge, UK), CD63, CD9 and CD81 (System Biosciences, Palo Alto, CA, USA), histone H2A.Z. (Mybiosource, San Diego, CA, USA), calreticulin (Mybiosource) and cytochrome C (ThermoFisher Scientific, Inc.). All the kits included standard proteins; therefore, the amounts of proteins and extracellular vesicles were determined based on a standard curve of each kit.

### qPCR

RNA was extracted from EVs using Trizol^{™} according to the instructions of the manufacturer and RNA was quantified by a nanodrop. RNA was made into cDNA through a reverse transcription (RT) process, and real-time PCR was performed according to the manual of the manufacturer using Taqman probes suitable for each miRNA and mRNA.

### EV labeling and uptake by cells

Purified EVs were stained with CFSE (5-(and-6)-Carboxyfluorescein Diacetate, Succinimidyl Ester) labeling dye (c-1157, Invitrogen) according to the manufacturer's instructions. Excess dye was removed by ultracentrifugation at 100.000 g for 1 hour. A human NSC cell line (ReNcells^{®}) was treated with the labeled EVs (0.4 µg/ml) and cultured for 24 hours. After treatment, cells were washed twice with PBS and stained using a conventional immunocytochemistry protocol. The cells were incubated overnight at 4°C with a mouse anti-SMA (1 : 100, Sigma aldrich) antibody. Thereafter, the cells were washed with PBS and cultured with a secondary antibody, DyLight-labeled anti-mouse IgG (1 : 200, 594 nm, Abcam) antibody. The cells were nuclear-stained using Vectashield^{™} with 1.5 µg/mL 4'-6' diamidino-2-phenylindole (DAPI) (Vector Laboratories), and then the cells were imaged using a confocal microscope (LSM 700, Carl Zeiss, Germany).

### Example 1. Isolation of extracellular vesicles through 3D culture of mesenchymal stem cells

### 1.1 Preparation of mesenchymal stem cells

Wharton's Jelly-derived mesenchymal stem cells (hereinafter referred to as WJ-MSC, Samsung medical center, Seoul, Korea) at passage 5 stage were received and cultured in a 37°C, 5% CO₂ incubator. A growth medium was used with an α-modified eagle's medium (α-MEM, GIBCO, NY, USA) containing 10% fetal bovine serum (FBS) (GIBCO, NY, USA) and 50 µg/mL gentamicin (GIBCO, NY, USA). WJ-MSCs of passage 6 stage were used to prepare a 3D spheroid-type cell aggregate.

### 1.2 Production of 3D spheroid-type cell aggregate culture medium

The WJ-MSCs of passage 6 stage prepared in Example 1.1 were washed with PBS, treated with trypsin (TrypLETM Express, GIBCO, NY, USA) and reacted in a CO₂ incubator for 5 minutes. Thereafter, a new serum-free medium was added to neutralize trypsin and the cells were restored to obtain a cell pellet using a centrifuge. Then, a new serum-free medium was added to prepare a cell suspension, and the cells were counted. After cell counting, 60 ml of the cell suspension was uniformly dispensed in a microarray including 69,000 microwells coated with 2-methacryloyloxyethyl phosphorylcholine polymer (MPC) with a diameter and depth of 500 µm × 200 µm, respectively, to be a density of 400 cells/well, and maintained in a static state to induce the spontaneous formation of spheroid-type cell aggregates, and cultured in a CO₂ incubator at 37°C for a total of 4 days to prepare a 3D spheroid-type cell aggregate culture medium (hereinafter, referred to as a 3D-static-spheroid culture medium).

### 1.3 Isolation of extracellular vesicles derived from 3D spheroid-type cell aggregate

The 3D-static-spheroid culture medium prepared in Example 1.2 was collected, centrifuged at 2,500 g for 10 minutes, and filtered with a 0.22 µm syringe filter to remove cell debris. Thereafter, the 3D-static-spheroid culture medium passed through 300 kDa of a hollow fiber membrane (Pall, NY, USA) using a tangential flow filtration (TFF) system to remove proteins, and extracellular vesicles were first isolated and purified once more with physiological saline to obtain high-purity 3D-static-spheroid-derived extracellular vesicles (hereinafter, 3D-static-spheroid EVs) of the present invention.

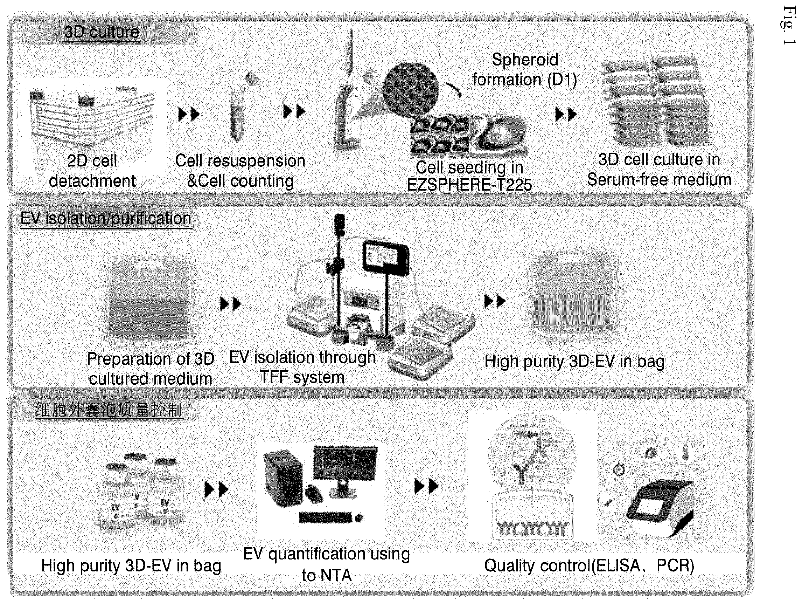

The processes of Examples 1.1 to 1.3 were schematically illustrated in FIG. 1.

### Example 2. Analysis of characteristics of 3D spheroid-type cell aggregate

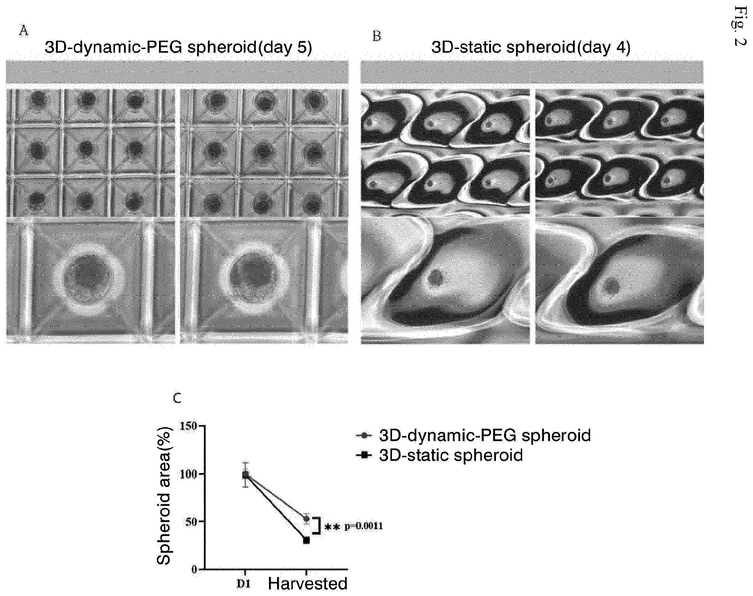

The characteristics of the 3D-spheroid-type cell aggregate (hereinafter, referred to as 3D-static-spheroid) present in the 3D-static-spheroid culture medium prepared in Example 1.2 were analyzed. As an experimental group, the 3D-static-spheroid culture medium prepared in Example 1.2 was used, and as a control group, a 3D mesenchymal stem cell spheroid-type cell aggregate (hereinafter referred to as 3D-dynamic-PEG spheroid) culture medium prepared by culturing mesenchymal stem cells for 5 days was used according to a dynamic 3D cell culture method disclosed in Registered Patent (Application No. 10-2016-0053026, method for producing stem cell-derived extracellular vesicles). With an optical microscope, microwells containing each culture medium were observed, which were illustrated in FIGS. 2A and 2B, and changes in spheroid-type cell aggregate area after culture compared to the initial culture were illustrated in FIG. 2C.

As illustrated in FIG. 2, as a result of comparing the areas of the 3D-static-spheroid of the experimental group and the 3D-dynamic-PEG spheroid of the control group, depending on a characteristic in which cells were densely condensed to form spheroids, compared to 3D-dynamic-PEG spheroids, 3D-static-spheroids showed a statistically significant decrease in area compared to spheroids at the beginning of culture. (p = 0.0011).

### Example 3. Size analysis of 3D spheroid-type cell aggregate

The size distributions of the 3D-static-spheroid prepared in Example 1.2 and the 3D-dynamic-PEG spheroid prepared in Example 2 were measured, and based on the measured size distribution data, dispersion coefficient, skewness and kurtosis were analyzed.

The skewness is a parameter capable of determining the inclined direction and degree of the distribution from the trend of a median value, and indicates a distribution with a longer tail to the right as closer to 1, and indicates a distribution with a longer tail to the left as closer to -1. In the case of a normal distribution, the skewness is 0.

The kurtosis is a parameter that indicates the degree of sharpness of the data distribution, and if the value is positive, it means that a relatively large number of data points are accumulated in the central part, and if the value is negative, it means that relatively few data are accumulated in the central part. In the case of a normal distribution, the kurtosis is 0.

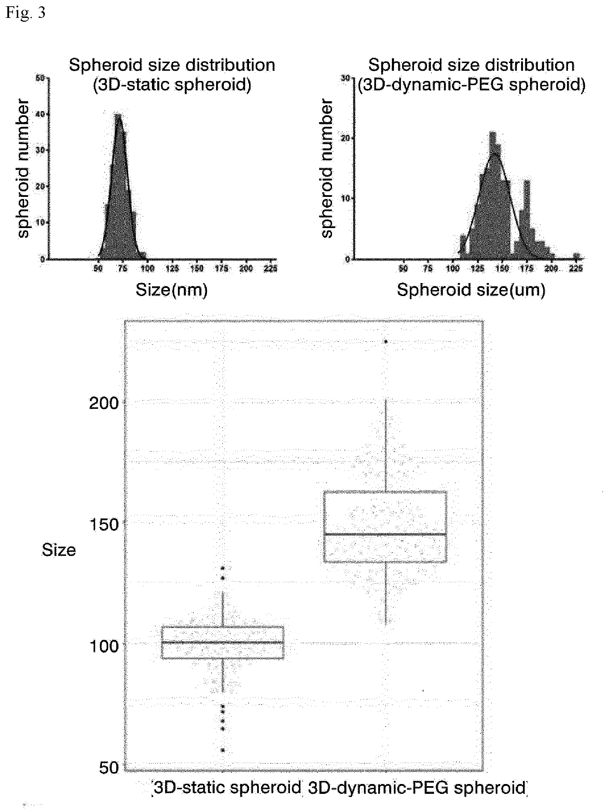

The measured size distribution data was illustrated in FIG. 3, and the results of analyzing the data were illustrated in Table 1.

**[Table 1]**

| | 3D-dynamic-PEG spheroid | 3D-static-spheroid |
|---|---|---|
| The number of spheroids | 154 | 155 |
| Size range | 108.4 - 225.0 um | 55.0 - 95.0 um |
| Size (mean ± SD) | 148.66 ± 20.89 um | 74.43 ± 7.756 um |
| Coefficient of variance | 14.1% | 9.59% |
| Skewness | 0.694 ± 0.195 | - 0.745 ± 0.195 |
| Kurtosis | 0.350 ± 0.389 | 1.799 ± 0.389 |

As illustrated in FIG. 3 and Table 1, it was confirmed that the average size of the 3D-static-spheroids was 74.43 um, and the coefficient of variance (CV) was 9.59%. In contrast, it was confirmed that the 3D-dynamic-PEG spheroids had an average size of 148.66 um and a coefficient of variance (CV) of 14.1%.

To compare and test the measured coefficient of variance, as a result of performing a Feltz and Miller's (1996) asymptotic test, a p value was calculated as 0.01765604, and as a result of performing a Krishnamoorthy and Lee's (2014) modified signed-likelihood ratio test, the p value was calculated as 0.01853969. Accordingly, in both the tests, it was confirmed that the size distributions of 3D-static-spheroids and 3D-dynamic-PEG spheroids showed statistically significant different patterns.

As a result of comparing the size distributions of the 3D-static-spheroids and the 3D-dynamic-PEG spheroids, it was confirmed that the kurtosis value of the size distribution of the 3D-static-spheroids was relatively large, and the size distribution of the 3D-static-spheroids of the present invention showed a tendency to be concentrated in the median values. From these results, it was confirmed that when the 3D spheroids were produced by the method for producing the 3D-static-spheroids of the present invention, it was possible to produce 3D spheroids having a relatively constant size.

### Example 4. Morphological analysis of extracellular vesicles derived from 3D spheroid-type cell aggregate

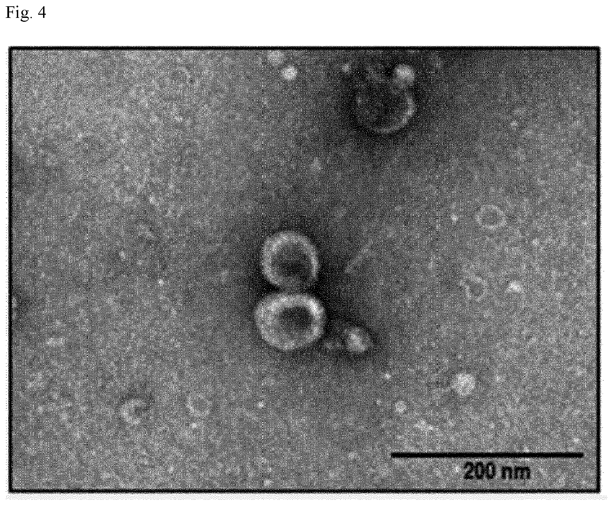

In order to observe the morphology of the 3D-static-spheroid EVs isolated in Example 1.3, the morphology was photographed using a transmission electron microscopy (TEM). Specifically, the 3D-static-spheroid EVs were fixed with 1% OsO₄ dissolved in a 0.1 M phosphate buffer (PB) for 2 hours. The EM grids were adsorbed onto the droplets of extracellular vesicles with a formvar side facing downward for 1 minute. Thereafter, the EM grids were blotted with filter paper and reacted with 2% uranyl acetate for 15 seconds. Excessive uranyl acetate was removed, and the EM grids were observed using a TEM (JEM-1011, JEOL, Japan), and the observed image was illustrated in FIG. 4.

As illustrated in FIG. 4, it was confirmed that the 3D-static-spheroid EV had a round shape, which was a typical shape of extracellular vesicles.

### Example 5. Concentration and size analysis of extracellular vesicles derived from 3D spheroid-type cell aggregate

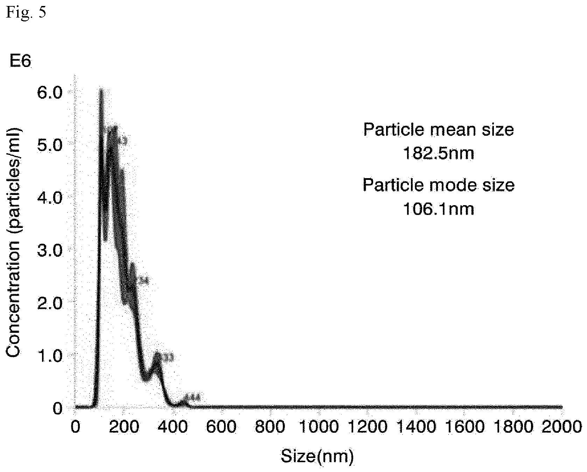

In order to confirm the concentration and size distribution of 3D-static-spheroid EVs isolated in Example 1.3, nanoparticle tracking analysis (NTA) was performed using NanoSight NS300 (Malvern, Worcestershire, UK). For optimal analysis, the 3D-static-spheroid EVs were pre-diluted in a vesicle-free phosphate buffer (PBS). The average size and concentration (particle/mL) were calculated by integrating three records, and the results were illustrated in FIG. 5.

As illustrated in FIG. 5, it was confirmed that the average particle diameter of the 3D-static-spheroid EV was 182.5 nm and the mode diameter was 106.1 nm.

### Example 6. Analysis of expression markers of extracellular vesicles derived from 3D spheroid-type cell aggregate

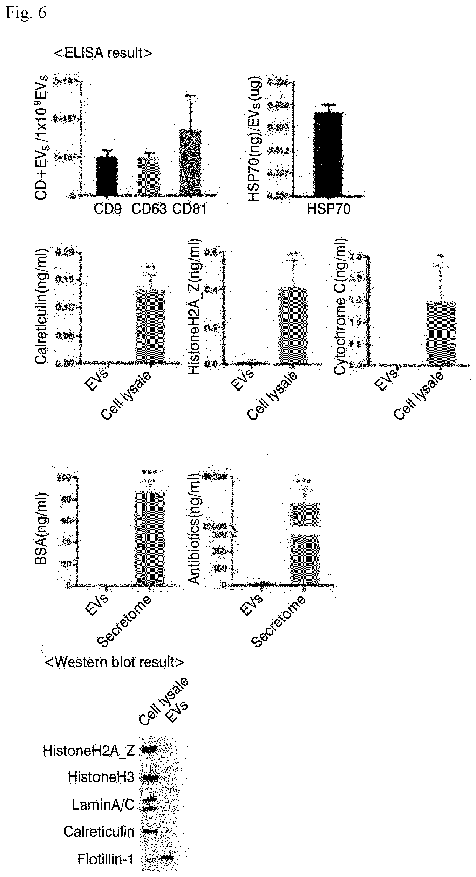

Experiments were performed to confirm expression markers of 3D-static-spheroid EVs isolated in Example 1.3. A cell lysate and a secretome were used as a control. The cell lysate was prepared by a method of washing 3D-static-spheroids with PBS, treating the 3D-static-spheroids with trypsin, restoring cells, and obtaining a cell pellet using a centrifuge of the restored cells. The secretome was prepared by a method of obtaining the cell pellet, isolating EVs from a supernatant of the culture medium through the process of Example 1.3, and obtaining the remaining culture secretome. For marker analysis, extracellular vesicles-specific positive markers CD9, CD63, CD81 and HSP70 were quantified using ELISA, and specific contaminant protein markers such as calreticulin, histone H2A.Z, cytochrome C, albumin, and antibiotics were quantified. In addition, histone H2A.Z, histone H3, lamin A/C, and calreticulin, which were specific contaminant protein markers of extracellular vesicles, were quantified using Western blot, and a positive marker of extracellular vesicles, flotillin-1 was quantified. The ELISA analysis result and the Western blot result were shown in FIG. 6.

As illustrated in FIG. 6, it was confirmed that 3D-static-spheroid EVs expressed all of the extracellular vesicles-specific positive markers CD9, CD63, CD81 and HSP70, and calreticulin, histone H2A.Z, histone H3, cytochrome C, albumin (BSA, bovine serum albumin), lamin A/C, and antibiotics, which were highly expressed contaminant protein markers in the cell lysate and the secretome, were not almost expressed. In particular, it was confirmed that flotillin-1 as a positive marker for extracellular vesicles, which was expressed very low in the cell lysate, was relatively highly expressed in 3D-static-spheroid EVs.

### Example 7. Analysis of production of extracellular vesicles derived from 3D spheroid-type cell aggregate

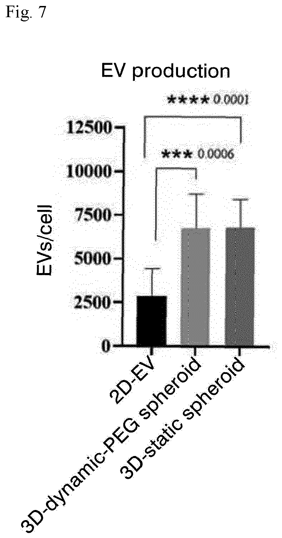

The experiment was performed to compare the productions of the 3D-static-spheroid EVs, which were the extracellular vesicles produced by the production method of Example 1, extracellular vesicles (3D-dynamic-PEG spheroid EVs) isolated from the 3D-dynamic-PEG spheroids of Example 2, and extracellular vesicles (2D-EVs) isolated from stem cells cultured by a conventional 2D culture method. The 2D-EVs were prepared by the following process. The stem cells cultured for 3 days in a cell stack were washed with PBS, replaced with a serum-free medium, and further cultured for 2 days. The culture medium was restored and the cell debris was continuously removed using centrifugation and a 0.2 µm filter. Thereafter, the culture medium passed through a hollow fiber membrane using a TFF system to remove proteins, and the EVs were first isolated, and purified once more with physiological saline to obtain high-purity 2D-EVs. The productions per cell derived from the prepared 3D-static-spheroid EVs, 3D-dynamic-PEG spheroid EVs, and 2D-EVs were compared and illustrated in Table 2 and FIG. 7.

**[Table 2]**

| | 2D-EV | 3D-dynamic-PEG spheroid EV | 3D-static-spheroid EV |
|---|---|---|---|
| Production (EVs/cell) | 2437 EVs/cell | 6791 EVs/cell | 6840 EVs/cell |

### Example 8. Expression microRNA (miRNA) analysis of extracellular vesicles derived from 3D spheroid-type cell aggregate

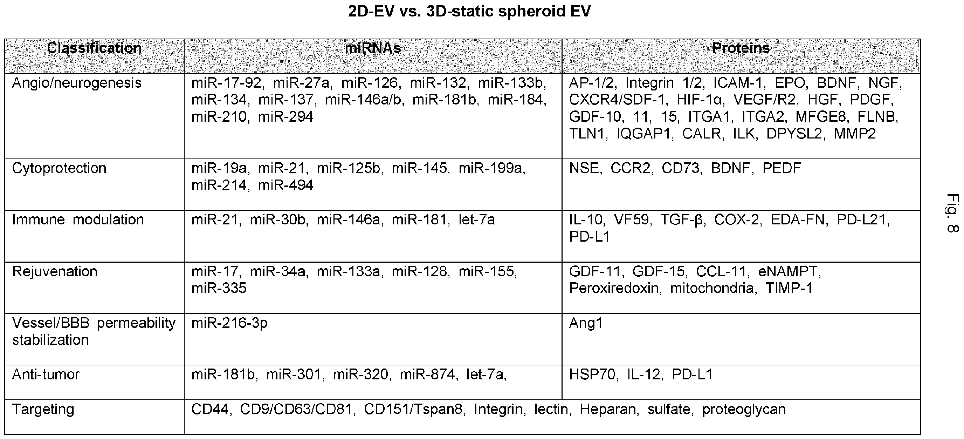

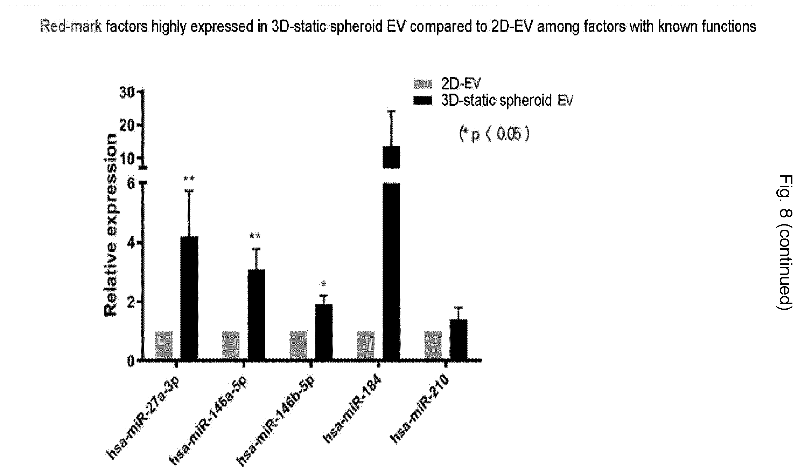

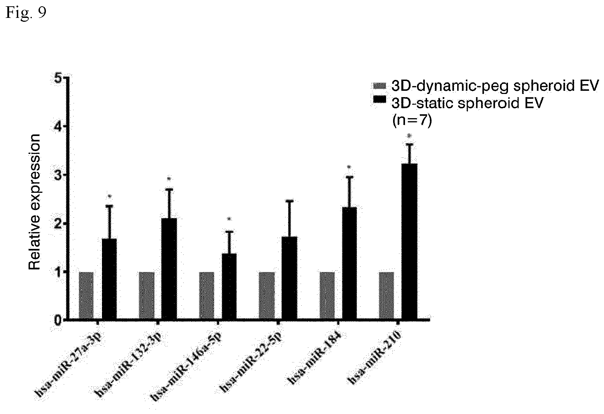

In order to confirm the differences in characteristics between 3D-static-spheroid EVs which were the extracellular vesicles produced by the production method of Example 1, 3D-dynamic-PEG spheroid EVs as extracellular vesicles isolated from 3D-dynamic-PEG spheroids of Example 2, and 2D-EVs prepared in Example 7, expression of miRNAs and protein expression were measured by qPCR. miRNAs and proteins highly expressed in the 3D-static-spheroid EVs compared to the 2D-EVs were illustrated in FIG. 8, and angiogenesis-related miRNAs and proteins highly expressed in the 3D-static-spheroid EVs compared to the 3D-dynamic-PEG spheroid EVs were illustrated in FIG. 9.

As illustrated in FIG. 8, it was confirmed that as compared with the 2D-EVs, the 3D-static-spheroid EVs highly expressed miR-146a, miR-27a, and miR-146b as miRNAs that exhibited the efficacy on angio/neurogenesis and immune regulation, and highly expressed integrin 1/2 and vascular endothelial growth factor/R2 (VEGF/R2) as proteins exhibiting the efficacy on angio/neurogenesis.

As illustrated in FIG. 9, it was confirmed that as compared with the 3D-dynamic-PEG spheroid EVs, the 3D-static-spheroid EVs highly expressed miR-146a, miR-27a, miR-132, miR-184 and miR-210 as miRNAs exhibiting the efficacy on angiogenesis.

From these results, it was confirmed that as compared to the 2D-EVs or 3D-dynamic-PEG spheroid EVs, the 3D-static-spheroid EVs, which were the extracellular vesicles produced by the production method of Example 1, were new extracellular vesicles with different miRNA and protein expression, and particularly, extracellular vesicles which may be clinically useful by highly expressing miRNAs related to angio/neurogenesis, immune regulation, rejuvenation or antitumor.

### Example 9. Confirmation of the angiogenesis promoting effect of extracellular vesicles derived from 3D spheroid-type cell aggregate

### 9.1 Comparison of expression of angiogenesis-related miRNA and Ephrin A3

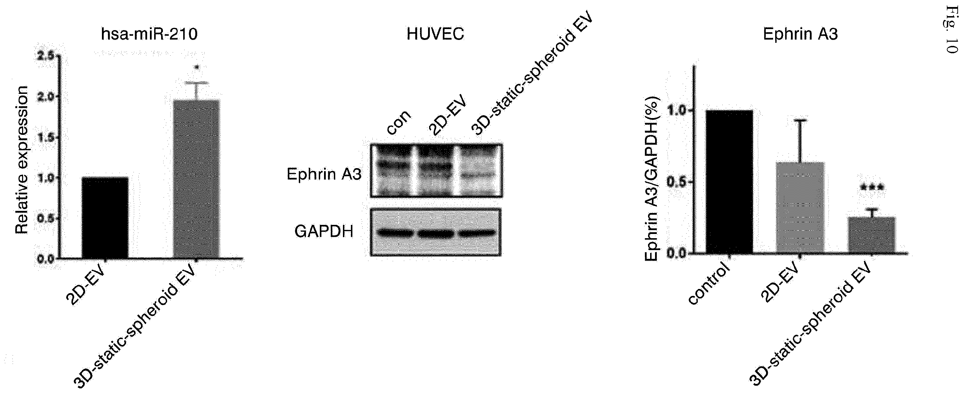

Through Example 8, it was confirmed that 3D-static-spheroid EVs, which were extracellular vesicles prepared by the preparation method of Example 1, were new extracellular vesicles with different expression patterns of miRNA and protein from existing 2D-EV or 3D-dynamic-PEG spheroid EVs. Accordingly, in order to confirm the usefulness of 3D-static-spheroid EVs in promoting angiogenesis, HUVEC cells, which were target cells, were treated with the 3D-static-spheroid EVs, and then changes in the expression of miRNA and eph-related receptor tyrosine kinase ligand 3 (Ephrin A3) related to the angiogenesis effect were confirmed through qPCR and Western blot, and the results were illustrated in FIG. 10.

As illustrated in FIG. 10, as the qPCR result, it was confirmed that the expression of miR-210 was significantly increased in the 3D-static-spheroid EV of the present invention compared to the existing 2D-EV. In addition, after the HUVEC was treated with the 3D-static-spheroid EV, it was confirmed that the protein expression of Ephrin A3 was significantly inhibited compared to a 2D-EV-treated group. The miR-210 was known to promote the migration of vascular endothelial cells and form a capillary-like structure, and targeted Ephrin A3. In other words, these results showed once again that the 3D-static-spheroid EV may induce effective angiogenesis.

### 9.2 Analysis of miRNA expression difference according to donor

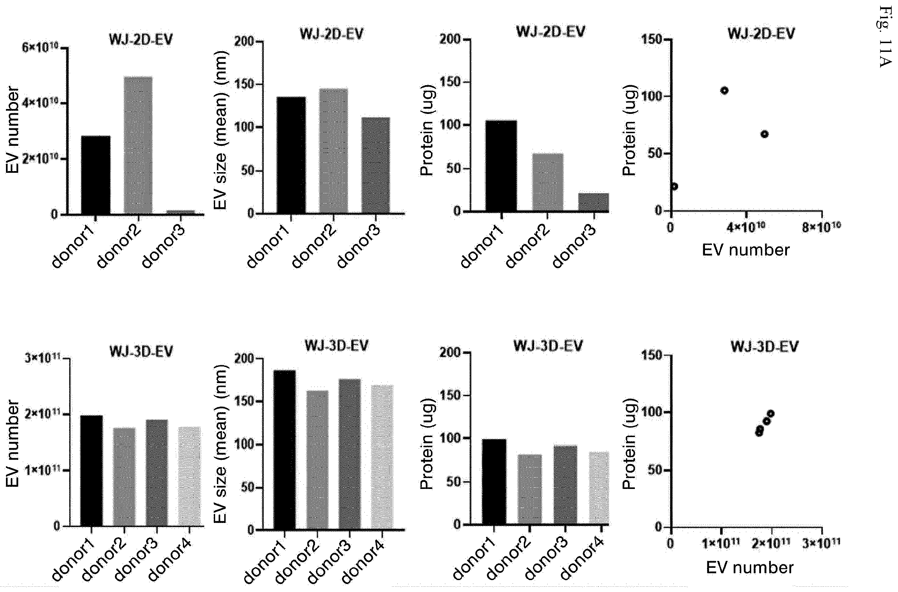

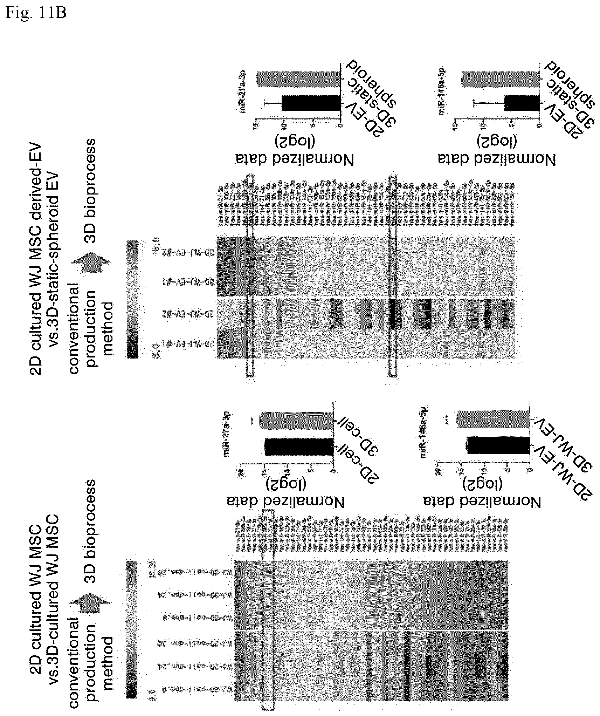

Stem cell therapy was known to have a problem of donor variation in which components varied depending on a donor. An experiment was performed to confirm whether the 3D-static spheroid EVs of the present invention exhibited a more consistent miRNA profile without a donor variation issue. Samples from each donor were received from the Samsung medical center (Seoul, Korea) and used. The numbers of EVs, EV protein amounts, and miRNA profiles produced by each donor were compared with each other in 2D-cultured WJ-MSCs and 3D-cultured WJ-MSCs of Example 1.1 and 3D-static-spheroid EVs, which were the extracellular vesicles produced by the production method of Example 1. miRNA was profiled by a Small RNA sequencing method, and the results were shown in FIG. 11A and FIG. 11B.

As illustrated in FIG. 11A, 2D-cultured WJ-MSC-derived 2D-EVs showed variations in the amount and size of EVs produced and the amount of EV-produced proteins depending on each donor, but the 3D-static-spheroid EVs showed consistent results without a large variation between donors.

In addition, as illustrated in FIG. 11B, it was confirmed that 2D-cultured WJ-MSCs and 2D-EVs had a large difference in miRNA composition produced depending on a donor, whereas 3D-cultured WJ-MSCs and 3D-static-spheroid EVs had a decreased difference depending on a donor. In particular, it was confirmed that the 3D-static-spheroid EVs showed a consistent miRNA profile without a difference depending on a donor, and at the same time, as compared with 2D-EVs, miR-27a-3p (1.5-fold), miR-146a-5p (2.3-fold), miR-210 (2.6-fold), and miR-132 (2.6-fold), which were miRNAs capable of exhibiting the angiogenesis effect, were more uniformly included. In addition, it was confirmed that miR-199a, miR-125b, miR-26a, let-7, miR-125a, miR-181b, and miR-92a, which were miRNAs related to angiogenesis, were expressed at uniform levels among respective donors.

These results indicated that the 3D-static-spheroid EVs may reduce a difference in therapeutic active ingredients according to a donor and exhibit better therapeutic effects.

As a result of combining all the results, the 3D-static-spheroid EVs of the present invention significantly increased angiogenesis-related miRNAs, miR-27a, miR-132, miR-146a, miR-146b, miR-184, and miR-210, and inhibited the Ephrin A3 protein expression compared to WJ-2D-EVs or 3D-dynamic-spheroid EVs. Accordingly, it was confirmed that the 3D-static-spheroid EVs of the present invention may be expected to have a better effect on angiogenesis than previously reported EVs, and may be an excellent therapeutic material by reducing differences depending on the donor.

### Example 10. Confirmation of expression changes of angiogenesis-related factors by treatment of extracellular vesicles derived from 3D spheroid-type cell aggregate

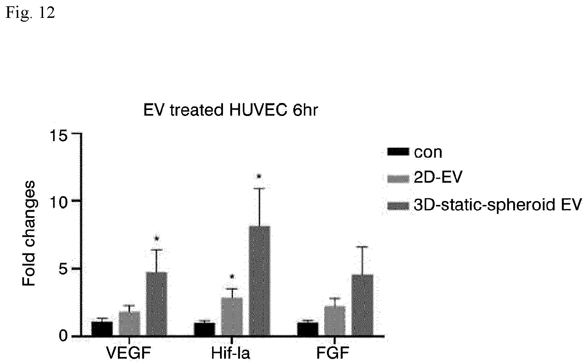

After vascular endothelial cells (HUVECs) were treated with 3D-static-spheroid EVs as the extracellular vesicles prepared by the preparation method of Example 1, and 2D-EVs as extracellular vesicles isolated from stem cells cultured by a conventional 2D culture method, changes in expression of angiogenesis-related factors, VEGF (vascular endothelial growth factor), Hif-1a (hypoxia-inducible factor 1-alpha), and FGF (fibroblast growth factor), were confirmed, and the results were illustrated in FIG. 12.

As illustrated in FIG. 12, it was confirmed that compared to cells treated with 2D-EVs, in the vascular endothelial cells (HUVECs) treated with 3D-static-spheroid EVs, the expression of VEGF (vascular endothelial growth factor), Hif-1a (hypoxia-inducible factor 1-alpha) and FGF (fibroblast growth factor) was increased.

The VEGF and Hif-1a were angiogenesis-related proteins, and the FGF was a protein factor that regulated biological functions related to cell proliferation, survival, and differentiation, and the 3D-static-spheroid EVs were extracellular vesicles for excellent clinical application in angiogenesis.

### Example 11. Confirmation of neuroangiogenic effect according to 3D-static-spheroid EV treatment

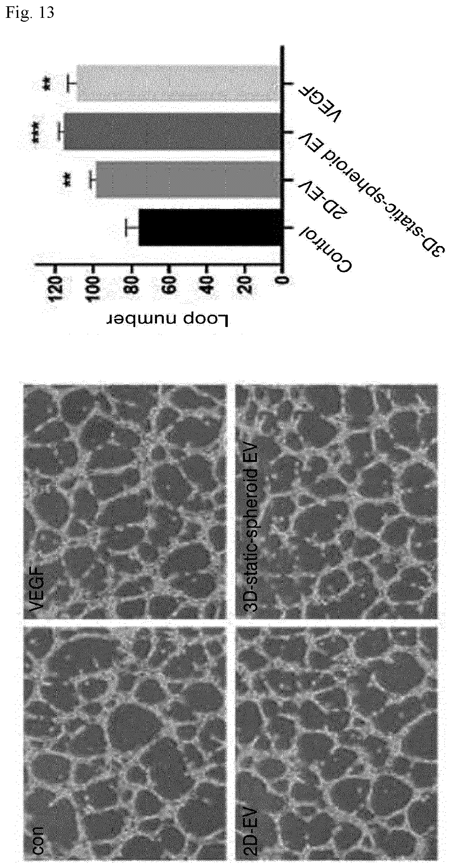

In order to confirm an effect of proliferation of neovascularization by extracellular vesicles obtained through 3D stem cell culture, HUVECs attached on Matrigel were treated with WJ-2D-EV and 3D-static-spheroid EV of the present invention, and then a tube formation effect was confirmed. Specifically, HUVECs were cultured in an M199 medium (Gibco) supplemented with 20% FBS, 5 U/mL heparin, and 3 ng/mL bFGF. The cells were inoculated on a growth factor-reduced Matrigel Matrix (BD Bioscience, MA, USA) from µ-Slides Angiogenesis (ibidi, Graefelfing, Germany) at a density of 1.7 × 10⁴ cells, and the tube was formed for 4 hours in a humidifying chamber under a 37°C and 5% CO₂ environment. Images were taken on a phase-contrast microscope (Olympus), and the number of tubular structures was quantified in a microscope field (4× magnification) using ImageJ software, and the results were illustrated in FIG. 13.

As can be seen in FIG. 13, it was confirmed that in the experimental group treated with the 3D-static-spheroid EV of the present invention, tube formation was significantly increased compared to a group treated with VEGF, an angiogenesis-related protein, and had an excellent tube formation effect even compared to the WJ-2D-EV-treated group.

### Example 12. Effect of 3D-static-spheroid EV on stroke animal model

### 12.1 Preparation of stroke model

To prepare a stroke animal model, a photothrombotic (PT) cerebral infarction model was prepared. PT stroke was induced in the right hand sensorimotor cortex of a mouse using adult male C57BL/6J mice (Orient Bio Inc., Seongnam, South Korea) of 20 to 25 g (8 to 12-week-old). Briefly, mice were anesthetized by intraperitoneal administration of a mixture of ketamine (100 mg/kg, Yuhan Corporation, Seoul, Korea) and xylazine (10 mg/kg, Rompun^{®}inj., Bayer, Berlin, Germany) and placed in a stereotaxic apparatus (KOPF Instruments, Tujunga, CA, USA). Using a scalpel, a midline incision was performed along the scalp from the eyes to the neck, and the skull was exposed after removing the periosteum. A Rose Bengal solution (30 mg/kg, saline 10 mg/mL, Sigma-Aldrich, St. Louis, MO, USA) was administered intravenously through the jugular vein, and after 5 minutes, a 100 mW, 532 nm diode-pumped solid-state green laser (Dongwoo Optron Co., Ltd., Gwangju) provided with a 3 mm-diameter illumination was positioned at 2.5 mm lateral to bregma. The laser was activated for 15 minutes in a region of interest (ROI) and the incision site was sutured using 6-0 monofilament suture. During surgery, rats were maintained at a body temperature of 37.0 to 37.5°C.

### 12.2 Confirmation of lesion improving effect of stroke model

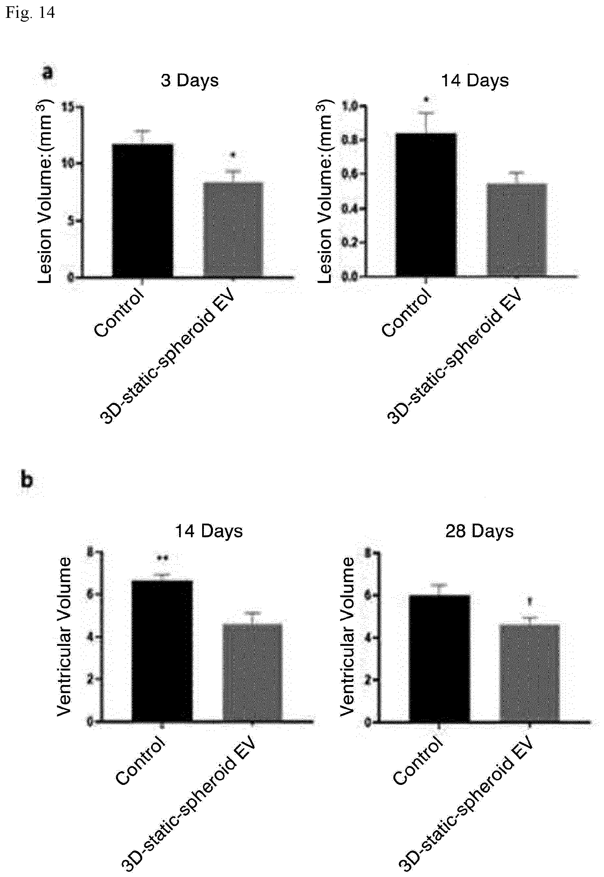

After vascular injection of PBS as a control group or 3D-static-spheroid EVs as an experimental group at 6 × 10⁸ EV/mouse into the stroke animal model prepared in Example 12.1, after 3 days (PBS group, n = 20; EV group, n = 23), 14 days (PBS group, n = 8; EV group, n = 10), and 28 days (PBS group, n = 8; EV group, n = 6), MRI T2-weighted images (T2WI) were taken to compare changes in cerebral infarction lesions and ventricular volumes in the experimental and control groups. The results were illustrated in Table 3 and FIG. 14.

**[Table 3]**

| | Cerebral infarction lesion | | Ventricular volume | |
|---|---|---|---|---|
| | **3 day** | **14 days** | **14 days** | **28 days** |
| Control group | 11.72 ± 1.17 | 0.84 ± 0.12 | 6.63 ± 0.28 | 6.00 ± 0.49 |
| 3D-static-spheroid EV-treated group | 8.39 ± 0.93* | 0.54 ± 0.07* | 4.60 ± 0.52** | 4.64 ± 0.32† |

| | | | | |
|---|---|---|---|---|
| Control group vs. 3D-static-spheroid EV-treated group, ↑p value = 0.052 *p value < 0.05; **p value < 0.01 | | | | |

As can be seen in Table 3 and FIG. 14, it was confirmed that the cerebral infarction lesion and the ventricular volume in the experimental group injected with the 3D-static-spheroid EV were significantly reduced compared to the PBS-treated control group.

### 12.3 Confirmation of angiogenesis effect through immunohistochemical analysis

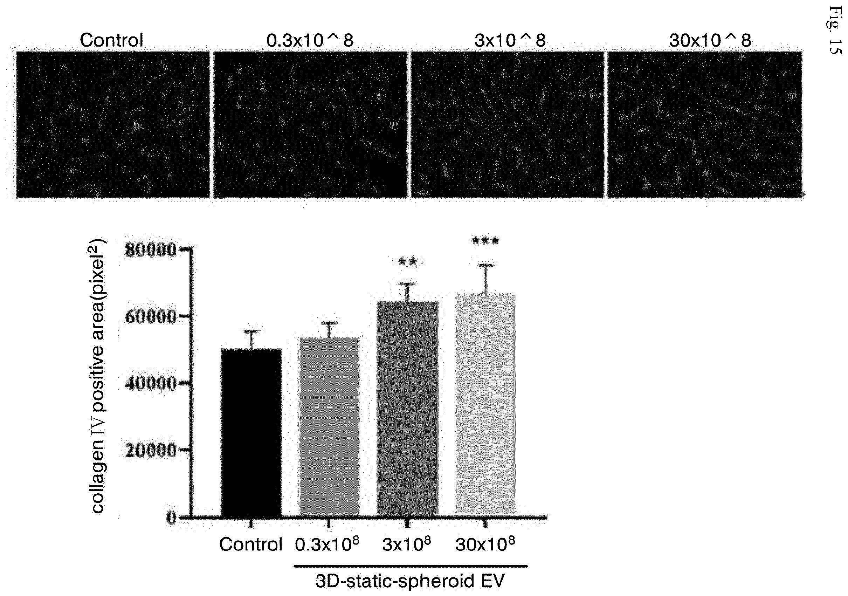

Immunohistochemical analysis was performed to confirm whether neural blood vessels could be effectively generated by injecting the 3D-static-spheroid EV of the present invention into a stroke animal model. The stroke animal model prepared in Example 12.1 was injected intravenously with PBS as the control group or 3D-static-spheroid EV as the experimental group at a rate of 0.3 × 10⁸ EV/mouse to 30 × 10⁸ EV/mouse, and after four weeks of stroke, immunofluorescence analysis was performed on brain tissue using collagen IV, a positive vascular marker, to confirm the number of blood vessels. The results were illustrated in FIG. 15.

As illustrated in FIG. 15, angiogenesis was confirmed in the stroke animal model injected with the 3D-static-spheroid EVs of the present invention compared to the PBS-treated control group, which was confirmed to be dependent on the number of injected EVs.

### 12.4 Confirmation of angiogenesis effect through behavioral analysis

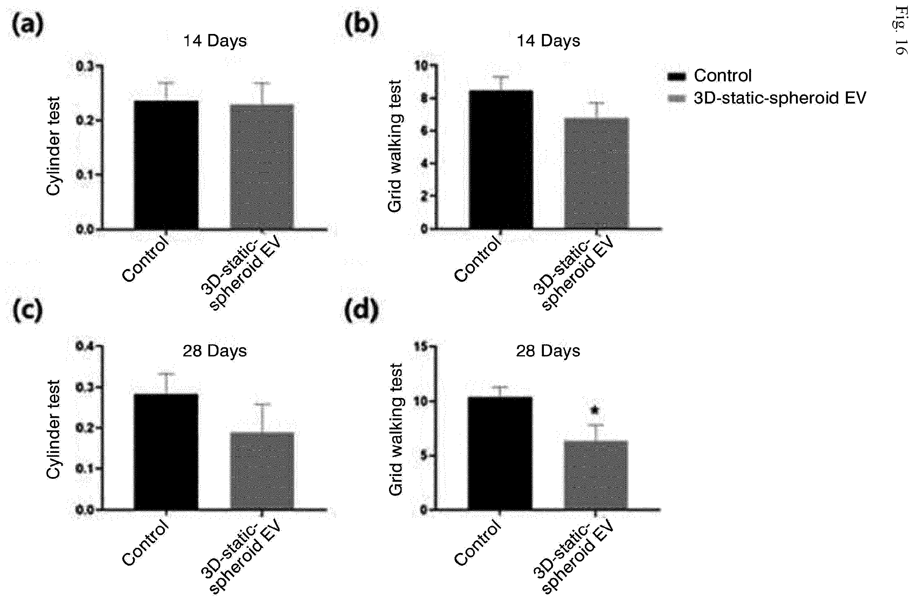

It was confirmed that angiogenesis was effectively induced by injecting the 3D-static-spheroid EVs of the present invention into a cerebral infarction animal model. To determine whether the loss of motor function in the cerebral infarction animal model was restored by angiogenesis, a forelimb asymmetry test (cylinder test) and a foot defect test (grid walking test) were performed. More specifically, 3D-static-spheroid EVs (6 × 10⁸ EV/mouse) were injected into the blood vessels of rats in the prepared photothrombotic cerebral infarction model, and at 14 days (PBS group, n = 8; 3D-static-spheroid EV treatment group, n = 10), and 28 days (PBS group, n = 8; 3D-static-spheroid EV treatment group, n = 6), the analysis was performed, and the results were illustrated in FIG. 16.

As illustrated in FIG. 16, it was confirmed that when the 3D-static-spheroid EV of the present invention was injected, both the cylinder test index and the grid walking test index were improved. These results showed that the 3D-static-spheroid EV treatment of the present invention may induce angiogenesis in the stroke animal model, and as a result, the impaired motor function may be improved.

### Example 13. Verification of 3D-static-spheroid EV effect according to preparation conditions

### 13.1 Experimental methods and conditions

Through Examples above, the excellent effect of the 3D-static-spheroid EVs of the present invention was confirmed. The preparation method of Example 1 was performed in the same manner, but to confirm whether the same effect was observed even in EVs prepared under different microwell specifications and cell number conditions, the cells were incubated by changing the cell count standard per microwell from 400 cells/well to 200 cells/well, or changing the microwell specification from 500 µm × 200 µm in diameter and depth to 500 µm × 600 µm, or a flat well with a diameter of 800 µm and no depth.

The experimental conditions changed from the preparation method of Example 1 were shown in Table 4 below.

**[Table 4]**

| | Experimental Example 1 | Experimental Example 2 | Experimental Example 3 |
|---|---|---|---|
| Number of cells per microwell (cell/microwell) | 400 | 400 | 200 |
| Diameter of microwell (µm) | 500 | 800 | 4-500 |
| Depth of microwell (µm) | 600 | / | 200 |

The WJ-MSCs of passage 6 stage prepared in Example 1.1 were washed with PBS, treated with trypsin (TrypLETM Express, GIBCO, NY, USA) and reacted in a CO₂ incubator for 5 minutes. Thereafter, a new serum-free medium was added to neutralize trypsin and the cells were restored to obtain a cell pellet using a centrifuge. Then, a new serum-free medium was added to prepare a cell suspension, and the cells were counted. After counting the cells, the cells were uniformly dispensed into microwells under the conditions shown in Table 4 above, maintained in a static state to induce spontaneous spheroid-type cell aggregate formation, and incubated in a CO₂ incubator at 37°C for a total of 4 days to prepare a 3D spheroid-type cell aggregate culture medium.

### 13.2 Confirmation of 3D spheroid-type cell aggregate morphology

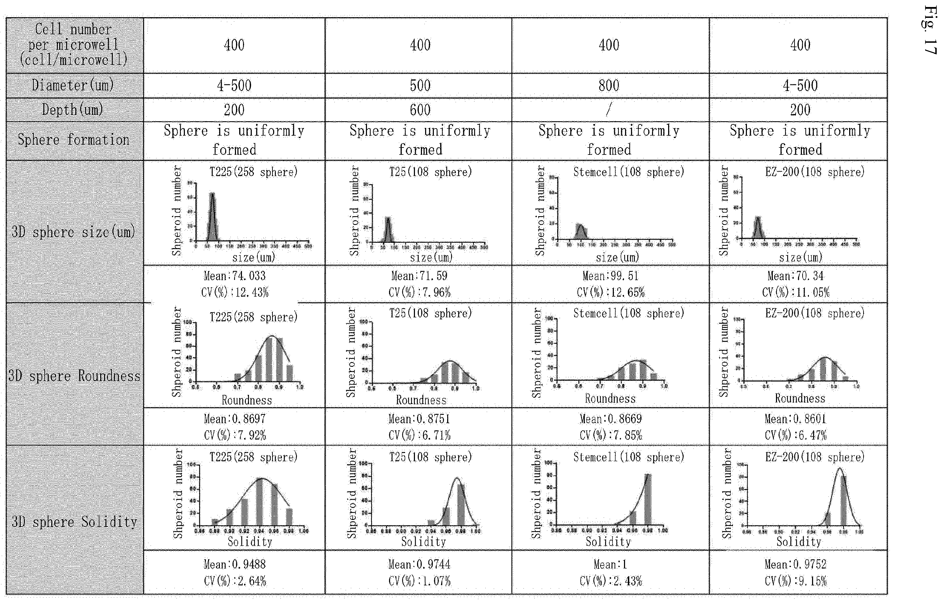

It was confirmed that spheroids were formed uniformly in the same manner as in Example 1 under the conditions of Experimental Examples 1 to 3, and the 3D spheroid-type cell aggregate culture medium was restored to obtain spheroid-derived extracellular vesicles by the method of Example 1.3. The size distribution of the obtained extracellular vesicles was measured, and the roundness and solidity of the spheroids were additionally confirmed, and the results were illustrated in FIG.17.

As illustrated in FIG.17, it was confirmed that the sizes of the 3D spheroid-type cell aggregates prepared in Experimental Examples 1 to 3 were within the range of 55 to 131 µm, which was the size range of the cell aggregate prepared in Example 1, and it was confirmed that the average values of the size distribution were 70.34 to 99.51 µm. In addition, as a result of confirming the roundness and solidity, it was confirmed that similarly to the average roundness value of 0.8697 (CV 2.64%) of the 3D spheroid-type cell aggregate of Example 1, the spheroid-type cell aggregates of Experimental Examples 1 to 3 also had the Roundness values of 0.8751, 0.8669, and 0.8601, respectively. In addition, it was confirmed that similarly to the average Solidity value of 0.9488 (CV 2.64%) of the 3D spheroid-type cell aggregate of Example 1, the spheroid-type cell aggregates of Experimental Examples 1 to 3 also had the average Solidity values of 0.9744, 1, and 0.9752, respectively.

These results showed that even if the number of cells per microwell was changed to 200 or 400 and the diameter of the microwell was changed to 400 to 800, the 3D spheroid-type cell aggregate with a similar shape may be effectively formed through the method of Example 1.

### 13.3 Comparison of miRNA expression patterns of 3D spheroid-type derived EVs

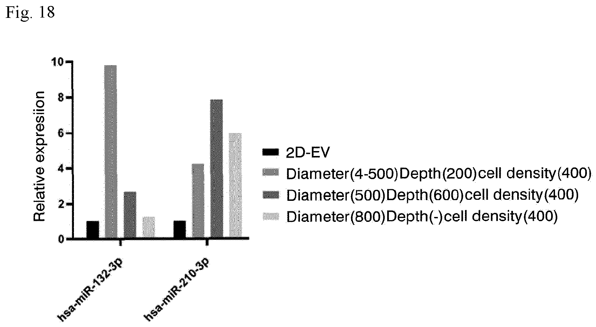

Since it was confirmed that spheroid-type cell aggregates having a similar shape to Example 1 could be prepared even under the conditions of Experimental Examples 1 to 3, additionally, extracellular vesicles were isolated and obtained from the spheroid-type cell aggregates by the method of Example 1.3, and it was confirmed whether the isolated and obtained EVs also showed the same miRNA expression patterns. Comparison of the miRNA expression patterns was confirmed using extracellular vesicles derived from spheroid-type cell aggregates prepared by the methods of Experimental Examples 1 and 2 under different microwell conditions. Analysis of expressed miRNA was confirmed through the same qPCR method as in Example 8. The results were illustrated in FIG. 18. The miRNA expression pattern was compared with that of the 2D-EV prepared in Example 7.

As illustrated in FIG. 18, it was confirmed that even if the microwell conditions were changed to 500 and 800 µm in diameter, in the same manner as the EVs prepared in Example 1, the expression of miR-132 and miR-210, which were miRNAs effective in angiogenesis/neurogenesis and immune regulation, was significantly increased compared to the existing 2D-EVs. These results showed that EVs derived from the three-dimensional spheroid-type cell aggregate obtained by the method of the present invention using microwells with a diameter of 200 to 800 µm may commonly exhibit miRNA marker expression characteristics. Therefore, the EVs may all be referred to as 3D-static-spheroid EVs.

### 13.4 Confirmation of angiogenesis effect of 3D spheroid-derived EVs

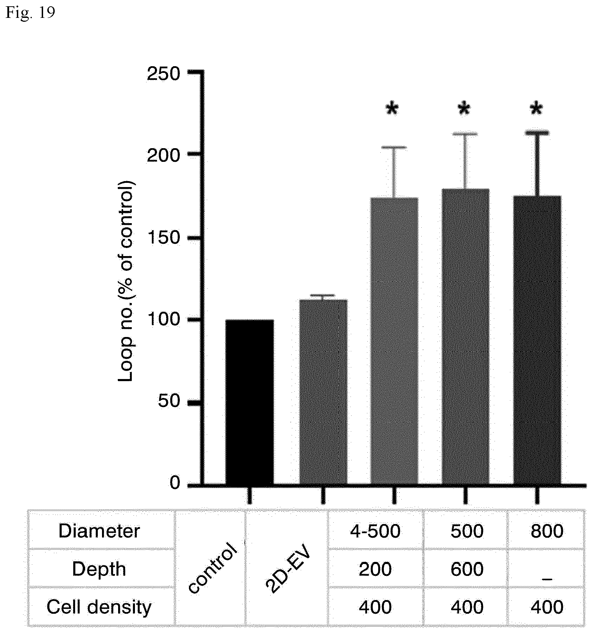

An experiment was performed to confirm the angiogenesis effect of 3D-static-spheroid EVs obtained through the method of the present invention using microwells with a diameter of 200 to 800 µm. As experimental groups, 3D-static-spheroid EVs prepared under the conditions of Experimental Examples 1 and 2 and Example 1, and 2D-EVs prepared in Example 7 were used, and the tube formation effect was confirmed in the same manner as in Example 11. The results were illustrated in FIG. 19.

As illustrated in FIG. 19, all 3D-static-spheroid EVs prepared under the conditions of Experimental Examples 1 and 2 and Example 1 showed a significantly excellent tube formation effect compared to the control group and 2D-EV.

In summary, the 3D-static spheroid EV of the present invention had high expression of angiogenesis-related miRNAs and angiogenesis factors, and had excellent angiogenesis ability *in vitro* and *in vivo* and the stroke improvement effect, and thus is expected to exhibit an excellent effect in preventing or treating various diseases that require vascular damage and angiogenesis.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A pharmaceutical composition for use in promoting angiogenesis comprising extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

2. The pharmaceutical composition for use according to claim 1, wherein the stem cells are at least one selected from the group consisting of mesenchymal stem cells, pluripotent stem cells, induced pluripotent stem cells and embryonic stem cells.

3. The pharmaceutical composition for use according to claim 1, wherein the 3D culture in step (a) is static culture.

4. The pharmaceutical composition for use according to claim 1, wherein the 3D culture in step (a) is performed by dispersing and culturing the mesenchymal stem cells in a microwell at a density of 200 to 600 cells/well.

5. The pharmaceutical composition for use according to claim 1, wherein the extracellular vesicles highly express at least one selected from the group consisting of miR-27a, miR-132, miR-146a, miR-146b, miR-184, and miR-210 as compared with extracellular vesicles derived from a spheroid-type cell aggregate obtained by 3D dynamic culture of mesenchymal stem cells and extracellular vesicles derived from mesenchymal stem cells obtained by 2D culture.

6. The pharmaceutical composition for use according to claim 1, wherein the extracellular vesicles highly express at least one selected from the group consisting of vascular endothelial growth factor (VEGF), hypoxia-inducible factor 1-alpha (Hif-1a), and fibroblast growth factor (FGF) as compared with extracellular vesicles derived from a spheroid-type cell aggregate obtained by 3D dynamic culture of mesenchymal stem cells and extracellular vesicles derived from mesenchymal stem cells obtained by 2D culture.

7. The pharmaceutical composition for use according to claim 1, wherein the pharmaceutical composition for promoting angiogenesis is for prevention or treatment of at least one selected from the group consisting of burns, ulcers, ischemia, arteriosclerosis, angina pectoris, myocardial infarction, cardiovascular diseases, cerebrovascular diseases, and alopecia.

8. The pharmaceutical composition for use according to claim 1, wherein the pharmaceutical composition for promoting angiogenesis is for promoting brain angiogenesis.

9. A food composition for use in promoting angiogenesis comprising extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

10. An *in vitro* composition for use in promoting angiogenesis comprising extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

11. A method for producing a composition for promoting angiogenesis comprising extracellular vesicles derived from a three-dimensional spheroid-type cell aggregate, comprising:
(a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

12. A method for promoting angiogenesis comprising:
(a) preparing a three-dimensional spheroid-type cell aggregate by 3-dimensionally (3D) culturing stem cells in a microwell with a diameter of 200 to 800 µm and a depth of 100 to 1000 µm;
(b) preparing extracellular vesicles derived from the three-dimensional spheroid-type cell aggregate by isolating extracellular vesicles from the three-dimensional spheroid-type cell aggregate; and
(c) treating the extracellular vesicles derived from the three-dimensional spheroid-type cell aggregate prepared in step (b) to a subject in need thereof.
